# FASCICULE DE BREVET EUROPEEN

(11) **EP 1 628 695 B1**
(45) Date de publication et mention de la délivrance du brevet: **13.03.2019**
(21) Numéro de dépôt: 04767172.2
(22) Date de dépôt: 25.05.2004
(51) Int. Cl.: A61M 5/315, A61M 5/50, A61M 5/31

(54) **SERINGUE PRE-REMPLIE AVEC COIFFE ANTI-EFFRACTION**
VORGEFÜLLTE SPRITZE MIT KAPPE GEGEN UNERWÜNSCHTEN ZUGRIFF
PRE-FILLED SYRINGE WITH ANTI-TAMPER CAP

(30) Priorité: 26.05.2003 FR 0306334
(43) Date de publication de la demande: 01.03.2006
(73) Titulaire: Becton Dickinson France, 38800 Le Pont-de-Claix (FR)
(72) Inventeur: PEROT, Frédéric, F-38760 SAINT PAUL DE VARCES (FR); FELIX-FAURE, Catherine, F-38000 Grenoble (FR); GRIMARD, Jean-Pierre, F-38450 Vif (FR)
(74) Mandataire: Brédeville, Odile Marie
(86) Numéro de dépôt international: PCT/FR2004/001294
(87) Numéro de publication internationale: WO 2004/105840

(56) Documents cités:
- EP-A- 0 209 976
- EP-A- 0 511 402
- CH-A- 360 168
- DE-A- 4 331 137
- US-A- 4 531 940
- US-A- 4 979 943
- US-A- 5 318 547
- US-A- 5 925 032

## Description

La présente invention concerne un dispositif à usage médical, du type seringue pré-remplie comprenant un piston, ledit dispositif comprenant des moyens d'empêcher l'accès au piston ainsi que son retrait, une fois ledit dispositif rempli avec un liquide médicamenteux par exemple.

Dans le domaine des seringues pré-remplies prêtes à l'emploi, il a déjà été décrit des dispositifs permettant de savoir si la seringue a déjà été utilisée ou non. Ces dispositifs se rapportent généralement à l'extrémité distale de la seringue, c'est-à-dire à l'extrémité de la seringue située au niveau de l'aiguille. Il a ainsi été proposé de recouvrir l'aiguille par un capuchon qu'il faut briser avant de pouvoir utiliser la seringue.

Toutefois, dans le cas des seringues pré-remplies, il est utile de prévoir un dispositif empêchant l'accès au piston. Il est également utile de prévoir un dispositif empêchant le retrait du piston. En effet, il peut arriver que la tige d'actionnement du piston soit enlevée, et que le piston soit ensuite percé par une aiguille pour injecter dans la seringue un liquide ou un composant non prévu ou non autorisé. Puis la tige est remise en place sur le piston.

Au cours du processus de fabrication de ce type de seringues, ces dernières sont remplies sous atmosphère stérile, puis ensuite emballées et éventuellement stérilisées. C'est entre ces deux étapes, remplissage et emballage, qu'il convient que la chambre interne de ces seringues ne soit pas exposée ou que le médicament qu'elle contient ne soit pas contaminé.

Le document EP 0 738 517 décrit un clip ouvert qu'on enfile, alors que la tige d'actionnement du piston est déjà montée, sur la collerette située sur la partie proximale du corps de la seringue afin d'empêcher le déplacement et/ou le retrait du piston de la seringue. Toutefois, ce clip, du fait qu'il est ouvert, peut facilement être retiré puis replacé sur la collerette de la seringue. Il ne peut donc servir à empêcher l'accès au piston. Il n'empêche pas non plus le retrait du piston.

Le document EP 0 511 402 A1 décrit une seringue pré-remplie comprenant un corps tubulaire muni d'une collerette proximale, la seringue comprenant une coiffe détachable montée sur la collerette. Le document US 4, 531, 940 décrit un piston pour seringue hypodermique. Le document CH 306 168 décrit une seringue pré-remplie comprenant un corps tubulaire muni d'une collerette proximale, la seringue comprenant une coiffe fixe sur la collerette. US 5925032 A1 décrit une autre seringue avec un dispositif de protection.

D'autres systèmes dits « fermés » existent, mais le retrait du piston n'est empêché que lorsqu'il y a présence de la tige d'actionnement du piston.

La présente invention vise à remédier à ce problème en proposant une seringue pré-remplie munie d'un moyen permettant d'empêcher à la fois l'accès au piston ainsi que son retrait.

La présente invention porte sur un dispositif à usage médical, du type seringue pré-remplie prête à l'emploi, selon la revendication 1.

Le dispositif selon l'invention empêche l'accès au piston ainsi que son retrait. En effet, dans le dispositif selon l'invention, si l'on essaie d'avoir accès au piston, on tente de retirer la tige d'actionnement mais, du fait de la forme de l'ouverture centrale de la coiffe rigide qui forme un moyen d'arrêt dans le sens proximal de la tige d'actionnement, cette dernière est stoppée par cette coiffe rigide et le piston ne peut être retiré. Par ailleurs, du fait de la présence d'un bouchon constitué d'un matériau résistant au perçage avec une aiguille, l'accès au piston est impossible.

Dans la présente demande, on entend par extrémité distale d'une pièce l'extrémité la plus éloignée de l'utilisateur du dispositif et par extrémité proximale, l'extrémité la plus proche de l'utilisateur du dispositif.

Le bouchon est disposé librement en translation dans le corps tubulaire, entre le piston et l'extrémité proximale dudit corps.

Dans une forme de réalisation de l'invention, la tige d'actionnement est fixée à son extrémité distale sur le bouchon.

Dans une autre forme de réalisation, la tige d'actionnement n'est pas fixée au bouchon.

Avantageusement, il existe une interface d'appui entre le bouchon et le piston.

Dans une forme préférée de réalisation de l'invention, un moyen d'arrêt dans le sens proximal est fixé sur la tige d'actionnement, entre la coiffe et le bouchon. L'extrémité distale de la tige d'actionnement peut être solidaire du piston, par exemple par l'intermédiaire du bouchon.

Avantageusement, le bouchon et le piston sont fixés en translation, l'un sur l'autre.

Dans une forme de réalisation non conforme à l'invention, la coiffe est en aluminium et elle est sertie sur la collerette du dispositif.

Dans une autre forme de réalisation non conforme à l'invention, la coiffe est en acier inoxydable et elle clipsée de façon définitive sur la collerette.

Selon l'invention, la coiffe est en matière plastique.

Selon l'invention, la coiffe se présente sous la forme de deux moitiés de bague rigides encliquetables l'une dans l'autre, les deux moitiés de bague étant aptes à être fixées sur la collerette en l'emprisonnant, chaque moitié de bague comprenant une plateforme inférieure comprenant une ouverture semi-circulaire correspondant aux dimensions extérieures du corps tubulaire et une plateforme supérieure comprenant une ouverture semi-circulaire adaptée pour le passage libre en translation de la tige d'actionnement.

Dans une forme de réalisation de l'invention, les deux moitiés de bague sont symétriques.

Selon l'invention, les moyens d'encliquetage d'une moitié de bague dans l'autre sont situés à l'intérieur de chaque moitié de bague. De préférence, la bague résultant de l'encliquetage de l'une dans l'autre des deux moitiés de bague ne présente sur sa surface extérieure aucun moyen d'encliquetage ou de désencliquetage.

Le bouchon est constitué d'un matériau résistant au perçage avec une aiguille. De préférence, l'épaisseur et la nature du matériau du bouchon sont choisies de telle manière que le bouchon ne soit pas perçable avec une aiguille. De préférence, le matériau constituant le bouchon est choisi parmi les matières plastiques, les polymères polyoléfines, les polycarbonates et leurs mélanges.

Avantageusement, le bouchon est relié à l'extrémité distale de la tige d'actionnement par vissage ou encliquetage de ladite tige dans le bouchon.

Dans une forme de réalisation de l'invention, le bouchon n'est pas solidaire du piston.

Dans une autre forme de réalisation de l'invention, le bouchon est relié au piston par vissage dudit bouchon dans le piston.

Dans une forme de réalisation de l'invention, la tige d'actionnement est munie d'au moins une saillie dirigée dans un plan sensiblement radial par rapport à l'axe de la tige d'actionnement, cette saillie étant située entre le bouchon et l'extrémité proximale du corps tubulaire.

L'invention sera mieux comprise de la description qui suit, en référence au dessin annexé :
- la figure 1 est une vue en coupe d'un dispositif non conforme à l'invention, une fois rempli,
- la figure 2 est une vue en coupe, une fois rempli, selon une deuxième variante non conforme à l'invention,
- la figure 3 est une vue en coupe partielle montrant la liaison d'une tige d'actionnement et d'un bouchon utilisables dans un dispositif selon l'invention,
- la figure 4 est une vue en coupe partielle montrant la liaison d'une tige d'actionnement, d'un bouchon et d'un piston utilisables dans un dispositif selon l'invention,
- la figure 5 est une vue en perspective éclatée d'une coiffe d'un dispositif selon l'invention,
- la figure 6 est une vue en perspective de dessus d'une moitié de bague d'une coiffe selon la figure 5,
- la figure 7 est une vue en perspective de dessous d'une moitié de bague d'une coiffe selon la figure 5,
- la figure 8 est une vue en perspective d'une coiffe selon la figure 5 une fois fermée.

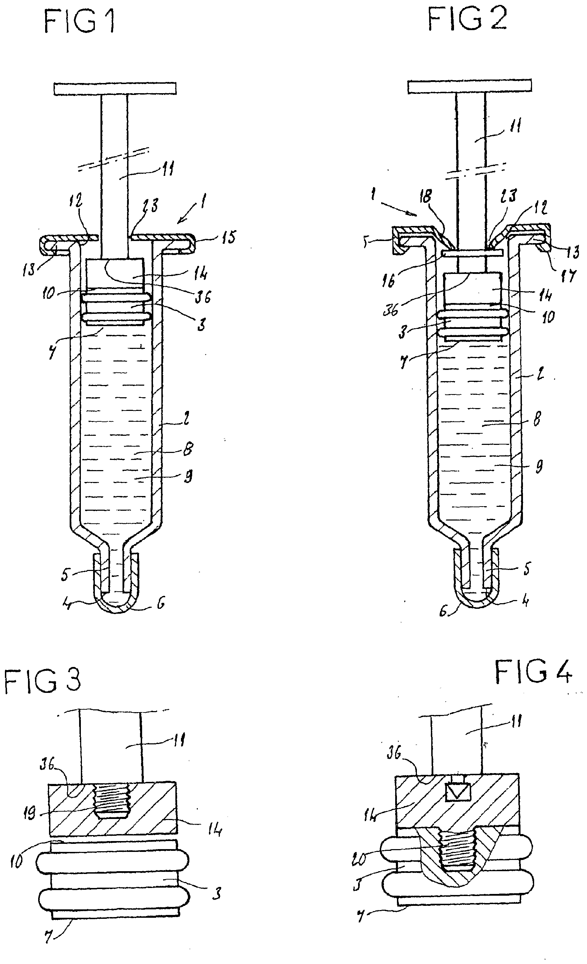

En se référant à la figure 1, est représenté un dispositif 1 comprenant un corps tubulaire 2 en verre et un piston 3 apte à coulisser à l'intérieur du corps tubulaire. Le corps tubulaire 2 comprend à son extrémité distale 4 un nez 5 de réception d'une aiguille, par exemple une aiguille creuse d'injection hypodermique (non représentée), fermé par un capuchon 6, et à son extrémité proximale 12 une collerette 13.

L'extrémité inférieure 7 du piston définit à l'intérieur du corps tubulaire 2 un volume utile interne 8 rempli par un liquide médicamenteux 9. Un bouchon 14, distinct du piston 3, est fixé à l'extrémité distale 36 d'une tige d'actionnement 11 dépassant de l'extrémité proximale 12 du corps tubulaire 2. Dans une forme non représentée, la tige d'actionnement 11 n'est pas fixée au bouchon 14. Ce bouchon 14 est disposé librement en translation à l'intérieur du corps 2, du côté proximal dudit piston 3, en appui contre ce dernier dans le sens distal. Il existe donc une interface d'appui 10 entre le bouchon 14 et le piston 3.

Une coiffe rigide 15 en aluminium est sertie sur la collerette 13 et présente une ouverture centrale 23 pour le passage libre en translation de la tige d'actionnement 11 dans le sens proximal.

Ainsi, lors du processus de fabrication du dispositif représenté sur la figure 1, si une personne tente de retirer la tige d'actionnement 11 du piston 3 du corps tubulaire après l'étape de remplissage du corps tubulaire 2 mais avant que le dispositif ne soit emballé, elle amène le bouchon 14 au contact de la coiffe 15 rigide. Cette coiffe arrête le bouchon 14 de la tige d'actionnement 11 et donc la tige d'actionnement 11 elle-même. Ainsi, même si on retire complètement la tige d'actionnement 11 du corps tubulaire 2, en la séparant du bouchon 14, ce dernier reste à l'intérieur du corps tubulaire 2 et empêche postérieurement l'accès au piston 3. Du fait de la présence du bouchon 14, il n'est pas possible de venir percer le piston 3, par l'introduction d'un moyen contondant par l'ouverture 23.

Sur la figure 2 est représenté une autre variante d'un dispositif non conforme à l'invention. Les références désignant les mêmes éléments dans les figures 1 et 2 ont été reprises. La tige d'actionnement 11 est munie de saillies radiales 16 situées l'une en regard de l'autre sur la partie de la tige d'actionnement se trouvant à l'intérieur du corps tubulaire 2. Ces saillies 16 formant moyens d'arrêt peuvent coopérer avec la coiffe 15 pour empêcher le déplacement du piston 3 vers l'extrémité proximale 12 du corps tubulaire au-delà d'une limite prédéterminée.

Selon cette deuxième variante, la coiffe 15 est en acier inoxydable et elle est clipsée de façon définitive sur la collerette 13 à l'aide d'un collet annulaire 17. La coiffe 15 comprend en outre des languettes radiales 18 s'étendant dans l'ouverture centrale 23, susceptibles d'arrêter en translation, et dans le sens proximal, les saillies 16.

Sur la figure 3 est représentée une tige d'actionnement 11 vissée à l'aide d'une vis ou partie filetée 19 dans le bouchon 14. Le piston 3 n'est pas relié au bouchon 14. Selon cette première variante utilisable dans un dispositif selon l'invention, il n'est donc pas possible à l'utilisateur de déplacer le piston 3 vers l'extrémité proximale du corps tubulaire 2 de la seringue. Il n'est pas possible non plus d'avoir accès au piston 3, même en cas de dévissage de la tige d'actionnement 11, du fait de la présence du bouchon 14 entre la coiffe 15 et le piston 3.

Sur la figure 4, est représentée une tige d'actionnement 11 fixée et encliquetée dans le bouchon 14. Selon cette deuxième variante utilisable dans un dispositif selon l'invention, le bouchon 14 est vissé sur le piston 3 à l'aide d'une vis ou partie filetée 20. Ainsi, l'extrémité distale de la tige d'actionnement 11 est solidaire du piston 3 par l'intermédiaire du bouchon 14.

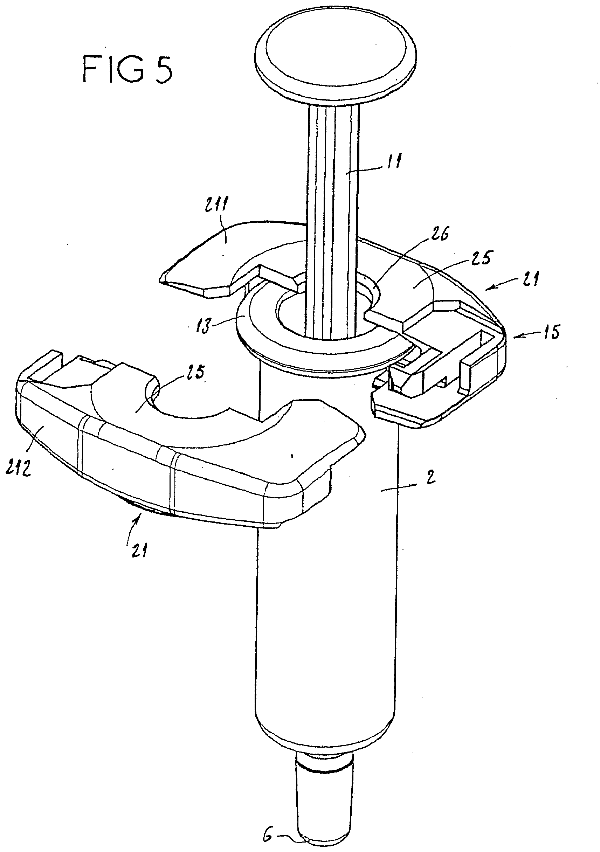

En se référant à la figure 5, est représentée une coiffe 15 comprenant une première moitié 211 de bague 21 fixée sur la collerette 13 de la seringue et une deuxième moitié 212 de bague 21 prête à être encliquetée dans ladite première moitié de bague 211.

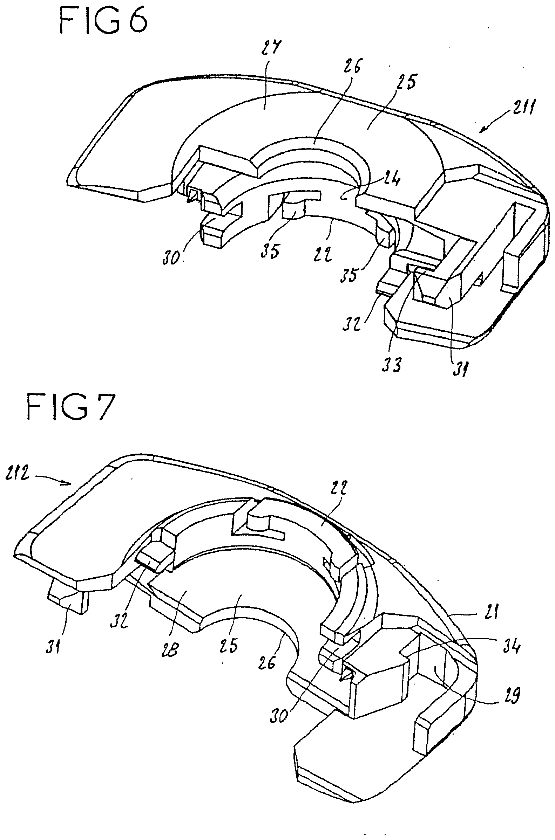

Comme il ressort de la figure 6, la moitié 211 de bague 21 comprend une plateforme inférieure 22 comprenant une ouverture semi-circulaire 24 correspondant aux dimensions externes du corps tubulaire 2. Cette moitié 211 de bague 21 comprend également une plateforme supérieure 25 comprenant une ouverture semi-circulaire 26 dont les dimensions permettent le passage libre en translation de la tige d'actionnement 11. La plateforme supérieure 25 comprend une face supérieure 27 et une face inférieure 28.

Comme il ressort des figures 6 et 7, chaque moitié 211 ou 212 de bague 21 comprend, situés entre sa plateforme inférieure 22 et sa plateforme supérieure 25, deux évidements distincts 29 et 30 et, de l'autre côté, deux téton distincts 31 et 32. Le téton 31 d'une première moitié 211 de bague 21 comprend un décrochage 33 apte à venir s'encliqueter dans un décrochage 34 de l'évidement 29 de la deuxième moitié 212 de bague 21. Le téton 32 d'une première moitié 211 de bague 21 est apte à venir s'encliqueter dans l'évidement 30 de la deuxième moitié 212 de bague 21.

Chaque moitié 211 ou 212 de bague 21 comprend également deux plots 35 permettant d'ajuster la plateforme inférieure 22 aux dimensions extérieures du corps tubulaire 2 de façon à ne pas compromettre la tenue de la bague sur la seringue.

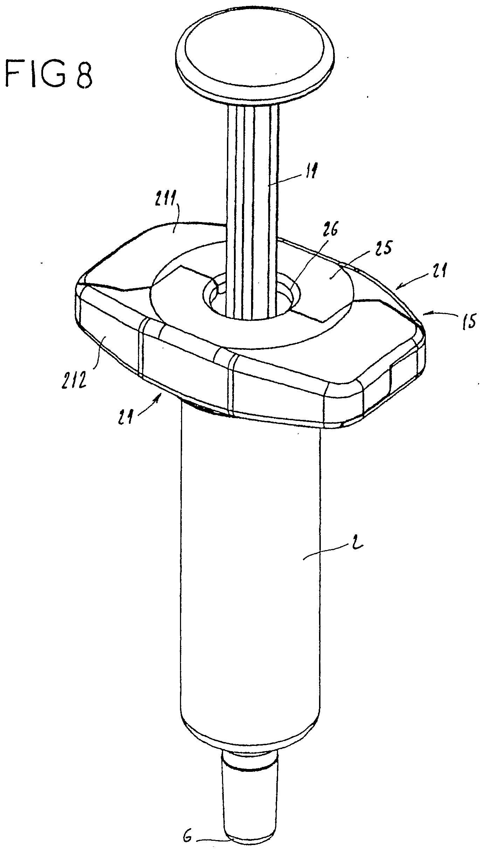

Sur la figure 8 est représentée la coiffe 15 des figures 5 à 7 pour laquelle les deux moitiés 211 et 212 de bague 21 sont encliquetées définitivement l'une dans l'autre, autour du corps tubulaire 2 et de part et d'autre de la collerette 13. Une fois la coiffe 15 fixée à demeure sur le corps tubulaire 2, il n'est pas possible d'accéder à l'intérieur dudit corps, sans détruire cette coiffe.

La présente invention n'est pas limitée aux formes d'exécution décrites dans la présente demande à titre d'exemples.

## Revendications

1. Dispositif (1) à usage médical, du type seringue pré-remplie prête à l'emploi, comprenant un corps tubulaire (2) comprenant à son extrémité distale (4) un nez (5) de réception d'une aiguille et à son extrémité proximale (12) une collerette (13), un piston (3) apte à coulisser à l'intérieur du corps tubulaire (2) et disposé dans une position initiale définissant avec le corps tubulaire (2) un volume utile interne (8), au moins partiellement rempli par un liquide médicamenteux (9), une tige d'actionnement (11) du piston s'étendant au-delà de l'extrémité proximale du corps tubulaire, une coiffe (15) rigide étant rapportée et fixée de façon définitive sur la collerette (13), et comportant une ouverture centrale (23) pour le passage libre en translation de la tige d'actionnement (11), et ladite coiffe (15) forme ou comporte des moyens d'arrêt dans le sens proximal de la tige d'actionnement (11),
le dispositif comprenant un bouchon (14) disposé librement en translation dans le corps tubulaire (2), entre le piston (3) et l'extrémité proximale (12) dudit corps, ledit bouchon (14) étant constitué d'un matériau résistant au perçage avec une aiguille,
la coiffe (15) étant en matière plastique et se présentant sous la forme de deux moitiés (211, 212) de bague (21) rigides encliquetables l'une dans l'autre, les deux moitiés de bague (21) étant aptes à être fixées sur la collerette (13) en l'emprisonnant, chaque moitié de bague (21) comprenant une plateforme inférieure (22) comprenant une ouverture semi-circulaire (24) correspondant aux dimensions extérieures du corps tubulaire (2) et une plateforme supérieure (25) comprenant une ouverture semi-circulaire (26) adaptée pour le passage libre en translation de la tige d'actionnement (11),
**caractérisé en ce que** les moyens d'encliquetage (30, 31, 32, 33, 34) d'une moitié de bague dans l'autre sont situés à l'intérieur de chaque moitié (211, 212) de bague (21), et **en ce que** les deux moitiés (211, 212) de bague sont séparées avant l'encliquetage.

2. Dispositif selon la revendication 1, **caractérisé en ce qu'**il existe une interface d'appui entre le bouchon (14) et le piston (3).

3. Dispositif selon la revendication 2, **caractérisé en ce qu'**un moyen (16) d'arrêt dans le sens proximal est fixé sur la tige d'actionnement (11) entre la coiffe (15) et le bouchon (14).

## Patentansprüche

1. Vorrichtung (1) mit medizinischem Gebrauch vom Typ vorgefüllte gebrauchsfertige Spritze, die einen röhrenförmigen Körper (2) umfasst, der an seinem distalen Ende (4) einen Ansatz (5) zum Aufnehmen einer Spritze, und an seinem proximalen Ende (12) einen Kragen (13) umfasst, einen Kolben (3) der geeignet ist, um in dem Inneren des röhrenförmigen Körpers (2) zu gleiten, und der in einer Anfangsposition angeordnet ist, die mit dem röhrenförmigen Körper (2) ein Nutzinnenvolumen (8) definiert, das mindestens teilweise von einer Arzneimittelflüssigkeit (9) gefüllt ist, wobei sich ein Betätigungsschaft (11) des Kolbens über das proximale Ende des röhrenförmigen Körpers hinaus erstreckt, eine starre Haube (15) angefügt und endgültig auf dem Kragen (13) befestigt ist und eine zentrale Öffnung (23) für das freie Durchgehen in Verschiebung des Betätigungsschafts (11) umfasst, und die Haube (15) Stoppmittel in die proximale Richtung des Betätigungsschafts (11) bildet oder umfasst,
wobei die Vorrichtung einen Stopfen (14) umfasst, der frei in Verschiebung in dem röhrenförmigen Körper (2) zwischen dem Kolben (3) und dem proximalen Ende (12) des Körpers angeordnet ist, wobei der Stopfen (14) aus einem Material besteht, das dem Durchstoßen mit einer Nadel standhält,
wobei die Haube (15) aus einem Plastikmaterial besteht und die Form von zwei starren Hälften (211, 212) eines Rings (21) aufweist, die ineinander einrastbar sind, wobei die zwei Ringhälften (21) geeignet sind, auf dem Kragen (13) befestigt zu sein, indem sie ihn einschließen, wobei jede Ringhälfte (21) eine untere Plattform (22) umfasst, die eine halbkreisförmige Öffnung (24) umfasst, die den Außenmaßen des röhrenförmigen Körpers (2) entspricht, und eine obere Plattform (25), die eine halbkreisförmige Öffnung (26) umfasst, die für das freie Durchgehen in Verschiebung des Betätigungsschafts (11) angepasst ist,
**dadurch gekennzeichnet, dass** die Einrastmittel (30, 31, 32, 33, 34) einer Ringhälfte in der anderen in dem Inneren jeder Hälfte (211, 212) des Rings (21) liegen, und dadurch, dass die zwei Ringhälften (211, 212) vor dem Einrasten getrennt sind.

2. Vorrichtung nach Anspruch 1, **dadurch gekennzeichnet, dass** eine Auflageschnittfläche zwischen dem Stopfen (14) und dem Kolben (3) existiert.

3. Vorrichtung nach Anspruch 2, **dadurch gekennzeichnet, dass** ein Mittel (16) zum Stoppen in die proximale Richtung auf dem Betätigungsschaft (11) zwischen der Haube (15) und dem Stopfen (14) befestigt ist.

## Claims

1. A ready-to-use pre-filled syringe type device (1) for medical use, comprising a tubular body (2) comprising at its distal end (4) a nose (5) for receiving a needle and at its proximal end (12) a flange (13), a plunger (3) adapted to slide inside the tubular body (2) and disposed in an initial position defining with the tubular body (2) an inner useful volume (8), at least partially filled with a medicinal liquid (9), a rod (11) for actuating the plunger extending beyond the proximal end of the tubular body, a rigid cap (15) being attached and fastened permanently on the flange (13), and including a central opening (23) for free passage in translation of the actuation rod (11), and said cap (15) forms or includes means for stopping the actuation rod (11) in the proximal direction,
the device comprising a plug (14) disposed so as to be free in translation in the tubular body (2), between the plunger (3) and the proximal end (12) of said body, said plug (14) being constituted of a material resistant to piercing with a needle,
the cap (15) being made of a plastic material and being in the form of two rigid ring (21) halves (211, 212) which may be snap-fitted into one another, the two ring halves (21) being adapted to be fastened on the flange (13) by trapping it, each ring half (21) comprising a lower platform (22) comprising a semi-circular opening (24) corresponding to the external dimensions of the tubular body (2) and an upper platform (25) comprising a semi-circular opening (26) adapted for the free passage in translation of the actuation rod (11),
**characterized in that** the snap-fit means (30, 31, 32, 33, 34) of one ring half into the other are located inside each ring (21) half (211, 212), and **in that** the two ring halves (211, 212) are separated before snap-fitting.

2. The device according to claim 1, **characterized in that** there is a bearing interface between the plug (14) and the plunger (3).

3. The device according to claim 2, **characterized in that** a means (16) for stopping in the proximal direction is fastened on the actuation rod (11) between the cap (15) and the plug (14).
